# EUROPÄISCHE PATENTANMELDUNG

(11) **EP 4 585 913 A1**
(43) Veröffentlichungstag der Anmeldung: **16.07.2025**
(21) Anmeldenummer: 24221110.0
(22) Anmeldetag: 18.12.2024
(51) Int. Cl.: G01N 27/04, G01N 27/22, G01N 33/38

(54) **FEUCHTESENSOR**

(30) Priorität: 09.01.2024 AT 52024
(71) Anmelder: e-4 Bauchemie GmbH, 6881 Mellau (AT)
(72) Erfinder: Albrecht, Richard, 6883 Au (AT)
(74) Vertreter: Torggler & Hofmann Patentanwälte - Rankweil

(57) **Zusammenfassung**

Feuchtesensor geeignet zur Restfeuchtemessung in einem Baustoff (2), insbesondere einem Estrich oder einem Beton, mit zumindest zwei Elektroden (3), bevorzugt zumindest drei Elektroden (3), und einer Messelektronik (4) zum Messen einer für die Restfeuchte indikativen Impedanz und/oder eines für die Restfeuchte indikativen Widerstands und/oder einer für die Restfeuchte indikativen Kapazität zwischen den zumindest zwei Elektroden (3), wobei die zumindest zwei Elektroden (3) innerhalb zumindest einer semi-permeablen Membran (5) angeordnet sind, wobei die zumindest eine semi-permeable Membran (5) eine höhere Durchlässigkeit für Wasserdampf aufweist als für Fremdstoffe, insbesondere größere Moleküle als Wasser und/oder Alkali-Ionen, welche die Messung der Impedanz und/oder des Widerstands und/oder der Kapazität verfälschen können.

## Beschreibung

Die vorliegende Erfindung betrifft einen Feuchtesensor geeignet zur Restfeuchtemessung in einem Baustoff, insbesondere ein Estrich oder ein Beton, gemäß den Merkmalen des Oberbegriffs des Anspruchs 1 sowie ein Verfahren zum Messen einer Restfeuchte in einem Baustoff.

Bekannt sind beispielsweise resistive Messungen in Verbindung mit Temperaturmessungen, die Rückschlüsse auf die Restfeuchte im Beton zulassen. Verwendet werden beispielsweise Sensoren, die in den Beton eingegossen werden oder nachträglich in ein Bohrloch im Beton eingebracht werden.

Verbesserungswürdig sind dabei die Messgenauigkeiten. Die resistive Messung wird nämlich neben der vorhandenen Feuchtigkeit auch von weiteren Faktoren beeinflusst. Konkret können Alkali-Ionen, die im Beton vorliegen, einen viel größeren Einfluss auf die elektrischen Messungen ausüben als Wasser.

Konkret unterliegen die in der GB 2213596 A, der DE 102009040942 A1 und der WO 2020/208471 A1 solchen Messungenauigkeiten.

Andere Ansätze machen sich beispielsweise ein feuchtedurchlässiges Kunststoffgehäuse zunutze, in welchem ein Luftfeuchtesensor angeordnet ist. Auch hier sind systematische Messfehler zu erwarten, weil letztlich nicht die Menge der Feuchtigkeit im Baustoff, sondern der umgebenden Atmosphäre gemessen werden, die nicht notwendigerweise einen direkten Zusammenhang aufweisen.

Darüber hinaus geht beispielsweise aus der WO 2017/100813 A1 eine "Feuchteindikatorvorrichtung" hervor, die eine Feuchteindikation ausgehend von einem mittels einer wasserdurchlässigen Membran abgeschirmten hygrischen Längenänderungselement - beispielsweise ein Stück Holz - auf rein mechanischem Wege bereitstellt.

Eine einfache und gleichzeitig genaue Messung von Restfeuchte in einem Baustoff ist daher offensichtlich wünschenswert.

Aufgabe der Erfindung ist es daher, einen Feuchtesensor und ein Verfahren zum Messen der Restfeuchtemessung in einem Baustoff bereitzustellen, die hinsichtlich der Genauigkeit verbessert ist, möglichst ohne die Komplexität der Messung zu erhöhen.

Diese Aufgabe wird in Bezug auf den Feuchtesensor durch die Merkmale des Anspruchs 1 gelöst, nämlich durch zumindest zwei Elektroden und einer Messelektronik zum Messen einer für die Restfeuchte indikativen Impedanz und/oder eines für die Restfeuchte indikativen Widerstands und/oder einer für die Restfeuchte indikativen Kapazität zwischen den zumindest zwei Elektroden, wobei die zumindest zwei Elektroden innerhalb zumindest einer semi-permeablen Membran angeordnet sind, und wobei die zumindest eine semi-permeable Membran eine höhere Durchlässigkeit für Wasserdampf aufweist als für Fremdstoffe, insbesondere größere Moleküle als Wasser und/oder Alkali-Ionen, welche die Messung der Impedanz und/oder des Widerstands und/oder der Kapazität verfälschen können.

Hinsichtlich des Verfahrens wird die Aufgabe durch die Merkmale des Anspruchs 16 gelöst, nämlich indem
- zumindest ein Feuchtesensor mit zumindest zwei Elektroden, die innerhalb zumindest einer semi-permeablen Membran angeordnet sind, verwendet wird, wobei die zumindest eine semi-permeable Membran eine höhere Durchlässigkeit für Wasserdampf aufweist als für Fremdstoffe, insbesondere Alkali-Ionen, welche die Messung einer Impedanz und/oder eines Widerstands und/oder einer Kapazität verfälschen können,
- der zumindest eine Feuchtesensor in den Baustoff eingegossen oder eingesetzt wird und
- die für die Restfeuchte indikative Impedanz und/oder der für die Restfeuchte indikativer Widerstand und/oder der für die Restfeuchte indikative Kapazität zwischen den zumindest zwei Elektroden gemessen wird.

Ein Grundaspekt der Erfindung ist es, die Impedanz und/oder den Widersand und/oder die Kapazität, welche jeweils für die Restfeuchte indikativ ist, mit geringerer systematischer Abweichung zu messen, indem die Elektroden von Fremdstoffen, insbesondere Alkali-Ionen, abgeschirmt werden, welche die Messung verfälschen.

Darunter, dass die zumindest zwei Elektroden innerhalb der zumindest einen semi-permeablen Membran angeordnet sind, kann verstanden werden, dass der Feuchtesensor einen Innenraum aufweist, in welchem die zumindest zwei Elektroden angeordnet sind und dass der Innenraum im Verwendungszustand mittels der zumindest einen semi-permeablen Membran vom Baustoff getrennt ist.

Dabei ist zu erwähnen, dass der Feuchtesensor auch noch andere Bauteile oder Elemente aufweisen kann, welche den Innenraum vom Baustoff trennen. Gemäß der Erfindung hat aber zumindest ein Teil des Innenraums eine Trennung vom Baustoff (nur) durch die semi-permeable Membran aufzuweisen, sodass die Feuchtigkeit aus dem Baustoff durch die semi-permeable Membran zu den zumindest zwei Elektroden gelangen kann.

In besonders bevorzugten Ausführungsformen ist die semi-permeable Membran zwischen zwei Lagen der zumindest einen semi-permeablen Membran angeordnet.

Der Innenraum wird im Folgenden und in den Ansprüchen auch kurz als das Innere des Feuchtesensors bezeichnet.

Da Impedanz-, Kapazitäts- und Widerstandsmessungen grundsätzlich mit vergleichsweise sehr hoher Genauigkeit durchgeführt werden können und aufgrund der Vermeidung von Verfälschungen der Messung durch eine semi-permeable Membran gemäß der Erfindung, kann insgesamt eine sehr genaue Messung der Restfeuchte in Baustoffen erzielt werden.

Beispielsweise kann dadurch besonders zuverlässig beurteilt werden, ob Estrich oder Beton so weit getrocknet (beispielsweise gemäß Herstellerangaben) sind, dass sie belastbar sind und/oder gesetzliche Normen für die Restfeuchte erfüllt sind (sogenannte "Belegreife"). Dies kann am Endgerät beispielsweise durch eine Ampelvisualisierung dargestellt werden.

Bei welchen Impedanzen (und gegebenenfalls Umgebungstemperaturen) welche Restfeuchten vorliegen und/oder ob Belegreife vorliegt, können Fachleute durch Experimente bestimmen.

Natürlich kann die Erfindung auch nachträglich verwendet werden, beispielsweise indem in bestehendem Baustoff ein Bohrloch gesetzt wird, worin dann ein erfindungsgemäßer Feuchtesensor und/oder ein erfindungsgemäßes Verfahren zum Einsatz kommen kann.

Ein weiterer Vorteil der Erfindung besteht darin, dass die zumindest eine semi-permeable Membran den Baustoff, dessen Restfeuchte ermittelt werden soll, kontaktiert. Dadurch wird die tatsächliche Restfeuchtigkeit gemessen, nicht eine Feuchtigkeit in der Atmosphäre in der Nähe des Baustoffs. Ebenso wenig wird die Messung durch andere Moleküle, wie beispielsweise Salze, verfälscht.

Unter der Messung der Restfeuchte wird insbesondere eine Messung einer Feuchtigkeitsverteilung über den Querschnitt (d.h. beispielsweise die Tiefe) im Baustoff verstanden. Das einfache Ermitteln von einer gemittelten Restfeuchte ist im Rahmen der Erfindung aber natürlich auch möglich.

Neben Estrich und Beton wären beispielsweise Holz, geschäumte Kunststoffe (bspw. Styropor) oder Lehm als Baustoffe zu nennen, deren Restfeuchte mittels der Erfindung erfasst werden kann.

In besonders bevorzugten Ausführungsformen der Erfindung wird eine Impedanzmessung durchgeführt, deren Realteil ausgewertet wird.

Die Impedanzmessung wird bevorzugt bei Frequenzen im Bereich von 50 Hz bis 50 kHz durchgeführt.

Besonders bevorzugt kann eine Frequenz im Bereich zwischen 1 kHz und 20 KHz sein.

In besonders bevorzugten Ausführungsformen kann eine Frequenz von 10 kHz verwendet werden.

Bei der angegebenen Frequenz und in den angegebenen Frequenzbereichen ist einerseits die Möglichkeit der Messung der Impedanz und/oder des Widerstands und/oder der Kapazität in leitfähigen Flüssigkeiten gegeben und andererseits werden unerwünschte Polarisationen vermieden, sodass bei der angegebenen Frequenz und in den angegebenen Frequenzbereichen mit den besten Messgenauigkeiten gerechnet werden kann.

Die semi-permeable Membran ist gemäß der Erfindung durchlässig für Wasserdampf, aber weniger durchlässig für Fremdstoffe, insbesondere größere Moleküle als Wasser und/oder Alkali-Ionen, welche die Messung verfälschen können.

Gemäß der Erfindung ist die semi-permeable Membran natürlich als solche zu verstehen, die während des für die Messung interessanten Zeitraums ihre Semi-Permeabilität mit ausreichender Güte beibehält.

Die zumindest eine semi-permeable Membran kann bevorzugt zumindest ein Polyurethan beinhalten oder aus zumindest einem Polyurethan bestehen.

Die semi-permeable Membran kann einlagig und/oder mehrlagig sein, in letzterem Fall beispielsweise ein mehrlagiges Laminat.

Die zumindest zwei Elektroden können in eine semi-permeable Membran eingeschlagen und/oder verklebt und/oder versteppt werden oder, wie erwähnt, zwischen zwei semi-permeablen Membranen angeordnet werden.

Die zumindest eine semi-permeable Membran kann in solchen Ausführungsformen randseitig verschweißt und/oder verklebt werden, sodass die zumindest zwei Elektroden zwischen den zwei Lagen der semi-permeablen Membran eingeschlossen ist. Diese Anordnung kann als "Messmembran" bezeichnet werden.

Dabei können Anschlussdrähte oder Anschlusskabel für die zumindest zwei Elektroden randseitig herausführen (beispielsweise durch die Verschweißung und/oder die Verklebung) und/oder über eine Ausnehmung in der Membran kontaktiert werden.

Neben der Kontaktierung über Anschlussdrähte, die aus dem Sensor herausgeführt werden, ist optional die Kontaktierung über eine Klemmkontaktierung möglich. Hierzu ist eine Ausnehmung in der Membran vorgesehen, welche das Leitmaterial der Elektrode freilegt. Die Messmembran wird bevorzugt zwischen Gehäuse und Platine positioniert und durch das Fixieren des PCB am Gehäuse wird das freigelegte Leitmaterial auf Kontaktflächen an dem PCB gedrückt und erzeugt dadurch den elektrischen Kontakt zum PCB.

Erfindungsgemäß müssen zumindest zwei Elektroden vorhanden sein, die vorzugsweise zwischen den zumindest zwei Lagen der zumindest einen semi-permeablen Membran angeordnet sind. In bevorzugten Ausführungsformen sind es zumindest drei Elektroden und besonders bevorzugt zumindest fünf Elektroden.

Durch das Erhöhen der Anzahl der Elektroden können gleichzeitig mehr Messwerte erhalten werden, wobei durch geschickte Datenauswertung eine höhere Genauigkeit und/oder eine bessere räumliche Auflösung der letztendlichen Ermittlung der Restfeuchte erzielt werden kann. Letzteres kann von besonderem Vorteil sein, weil beispielsweise Estrich im Regelfall inhomogen trocknet, was durch das Messen der Restfeuchte in verschiedenen Lagen messtechnisch erfasst werden kann.

Zu erwähnen ist, dass im Rahmen der Erfindung unter dem Eingießen des Feuchtesensors beispielweise auch das Umspritzen und/oder Laminieren des Feuchtesensors zu subsummieren ist.

Schutz begehrt wird darüber hinaus für eine Anordnung beinhaltend einen Baustoff und zumindest einen erfindungsgemäßen Feuchtesensor.

Schutz begehrt wird außerdem für die Verwendung der erfindungsgemäßen Feuchtesensoren oder einer erfindungsgemäßen Anordnung bei einem erfindungsgemäßen Verfahren.

Weitere vorteilhafte Ausführungsformen der Erfindung sind in den abhängigen Ansprüchen definiert.

Die zumindest zwei Elektroden kann bevorzugt zwischen zwei Lagen der zumindest einen semi-permeablen Membran angeordnet sein und vorzugsweise kann zwischen den Lagen der semi-permeablen Membran eine textile Zwischenlage angeordnet sein, in welche die zumindest zwei Elektroden eingearbeitet sind.

Bei der textilen Zwischenlage kann es sich bevorzugt um eine Zwischenlage handeln, welche ein saugfähiges Material beinhaltet oder daraus besteht. Dadurch kann sich in der Umgebung der zumindest zwei Elektroden eine Feuchtigkeit einstellen, welche jener im Baustoff möglichst gut entspricht.

Würde man beispielsweise nur gewisse Kunstfasern verwenden, die nur kapillar saugen, ist zu vermuten, dass sich insgesamt ein höherer Widerstand einstellt und dadurch stärkere Schwankungen auftreten. Grundsätzlich könnte die Erfindung aber auch mit solchen Zwischenlagen realisiert werden.

Beispielsweise kann es sich bei einem Material der Zwischenlage besonders bevorzugt um ein Polyester-Baumwoll-Mischgewebe und/oder anderes Material mit Baumwolle und/oder Viskose handeln, da diese gemeinhin als saugfähig eingestuft werden.

Die zumindest zwei Elektroden können bevorzugt als Drahtelektroden ausgebildet sein und/oder Garnelektroden ausgebildet sein und/oder aus Edelstahl bestehen.

Als Material für die zumindest zwei Elektroden können bevorzugt korrosionsbeständige leitende Materialien oder Mischungen solcher Materialien eingesetzt werden. Beispiele wären Edelstahl und/oder Carbon in Form von Filamenten und/oder Druckpasten.

Die zumindest zwei Elektroden können auf die textile Zwischenlage durch Sticken, Weben, Wirken, Stricken, Nähen, Steppen und/oder Drucken auf die Zwischenlage aufgebracht sein.

Es kann besonders bevorzugt ein Trägerkörper vorgesehen sein und die zwei Lagen der semi-permeablen Membran können samt der dazwischen angeordneten zumindest zwei Elektroden an einer Außenfläche des Trägerkörpers angeordnet sein, wobei die Außenfläche des Trägerkörpers bevorzugt eine Wabenstruktur beinhaltet.

Die Messelektronik kann bevorzugt in einem Inneren des Trägerkörpers angeordnet ist.

Der Trägerkörper kann bevorzugt eine zylindrische Grundform, vorzugsweise mit kreisförmiger Grundfläche, aufweisen. Natürlich können andere Grundflächenformen (bspw. oval, polygonal) ebenfalls verwendet werden.

Offene Seiten, bevorzugt obere und/oder untere Deckflächen, des Trägerkörpers können bevorzugt mittels eines Kunststoffs versiegelt sein.

Besonders bevorzugt können mehr als zwei, bevorzugt fünf, Elektroden vorgesehen sein, wodurch sich auf relativ einfache Weise eine Restfeuchteverteilung erfassen lässt.

Die zumindest zwei Elektroden können bevorzugt entlang einer Achse, bevorzugt äquidistant, beabstandet sein. Auch dies ist für eine besonders einfache Erfassung der Restfeuchteverteilung zielführend.

Die Achse, entlang der die zumindest zwei Elektroden beabstandet sind, kann besonders bevorzugt normal zu einer Oberfläche des Baustoffs ausgerichtet sein, wodurch die Restfeuchte in verschiedenen Tiefen im Baustoff erfasst werden kann.

Ein Temperatursensor und/oder ein Luftfeuchtesensor kann vorgesehen sein, welcher bevorzugt im Inneren des Trägerkörpers angeordnet ist.

Bevorzugt kann ein Kommunikationsmodul geeignet zur drahtlosen Kommunikation von Messwerten und/oder Restfeuchtewerten vorgesehen sein, welches bevorzugt im Inneren des Trägerkörpers angeordnet ist

Durch ein drahtloses Kommunikationsmodul kann die erfindungsgemäße Restfeuchtemessung ohne Zerstörung des Baustoffs erfolgen, wenn der erfindungsgemäße Feuchtesensor in den Baustoff eingegossen wird und darin verbleibt (verlorener Sensor).

Für die drahtlose Kommunikation kann beispielsweise mittels Bluetooth (insbesondere Bluetooth Low Energy) mit einem mobilen Endgerät kommuniziert werden. Natürlich kann die Erfindung grundsätzlich auch über andere Drahtlosprotokolle oder auch drahtgebunden verwirklicht werden.

Das drahtlose Übertragen der Messwerte und/oder von Ergebnissen der Messung(en) kann besonders bevorzugt von extern, insbesondere dem Endgerät, ausgelöst werden. Natürlich sind andere Ausführungsformen auch prinzipiell denkbar, wobei zum Beispiel festgesetzte zeitliche Übertragungspläne verwendet werden.

Es können beispielsweise die Impedanzmesswerte und/oder die Widerstandsmesswerte und/oder die Kapazitätsmesswerte und/oder die Temperaturmesswerte und/oder die Luftfeuchtemesswerte an das mobile Endgerät übertragen werden, welches dann, beispielsweise basierend auf einer Datenbank die zu ermittelnde Restfeuchte am Ort des Feuchtesensors ausgibt.

Alternativ oder zusätzlich kann der Sensor selbst basierend auf den Impedanzmesswerten und/oder den Widerstandsmesswerten und/oder den Kapazitätsmesswerten und/oder den Temperaturmesswerten und/oder den Luftfeuchtemesswerten auf die zu ermittelnde Restfeuchte schließen und nur oder zusätzlich diese an das mobile Endgerät übermitteln.

Die ermittelte Restfeuchte kann, wie erwähnt, direkt am mobilen Endgerät ausgegeben werden und/oder in eine Datenbank, beispielsweise in Form eines Computerservers, hochgeladen werden.

Zu den vorerwähnten Zwecken kann die Messelektronik, der Temperatursensor und/oder der Luftfeuchtesensor zum Datenaustausch mit dem Kommunikationsmodul und/oder miteinander ausgebildet sein.

Die Messelektronik kann batteriebetrieben sein.

In besonders bevorzugten Ausführungsformen kann in zumindest einer der Lagen der zumindest einen semi-permeablen Membran eine Ausnehmung vorhanden sein und die zumindest zwei Elektroden können mit einer Platine kontaktiert sein, indem die zumindest zwei Elektroden samt der zumindest einen semi-permeablen Membran im Bereich der Ausnehmung zwischen die Platine und den Trägerköper, vorzugsweise im Bereich eines Montagekörpers des Trägerkörper, geklemmt ist.

Weitere Vorteile und Einzelheiten der Erfindung ergeben sich aus den Figuren sowie der dazugehörigen Figurenbeschreibung. Dabei zeigen:
- Fig. 1: schematisch ein Ausführungsbeispiel einer erfindungsgemäßen Anordnung,
- Fig. 2: schematisch ein Ausführungsbeispiel eines erfindungsgemäßen Feuchtesensors,
- Fig. 3a bis 3d: schematisch ein Ausführungsbeispiel eines Trägerkörpers,
- Fig. 4a bis 4d: das Ausführungsbeispiel aus Fig. 3a bis 3d in einem teilweise zusammengebauten Zustand,
- Fig. 5a bis 5d: das Ausführungsbeispiel aus Fig. 3a bis 3d in verschiedenen Explosionsdarstellungen,
- Fig. 6: schematisch ein Ausführungsbeispiel einer Messmembran zusammen mit einer Messelektronik,
- Fig. 7: die Messmembran aus dem Ausführungsbeispiel aus Fig. 6 in einer teilweise geschnittenen Darstellung sowie
- Fig. 8: ein alternatives Ausführungsbeispiel einer Messmembran.

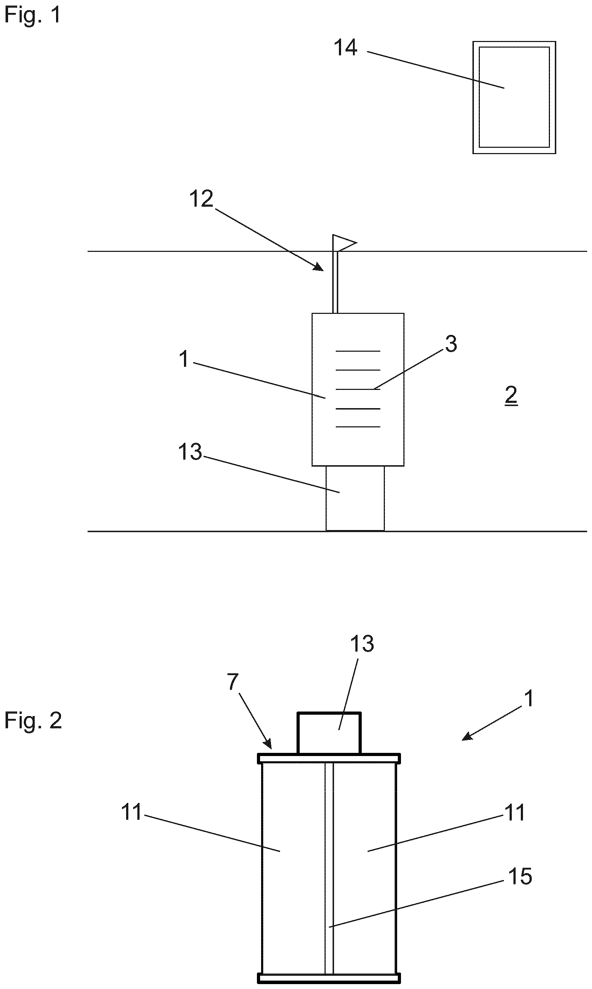

Fig. 1 zeigt schematisch ein Ausführungsbeispiel einer erfindungsgemäßen Anordnung mit einem Baustoff 2 und einem Feuchtesensor 1 gemäß der Erfindung.

Der Feuchtesensor 1 ist dabei in den Baustoff 2, beispielsweise ein Estrich oder Beton, eingebettet, in diesem Ausführungsbeispiel durch Eingießen.

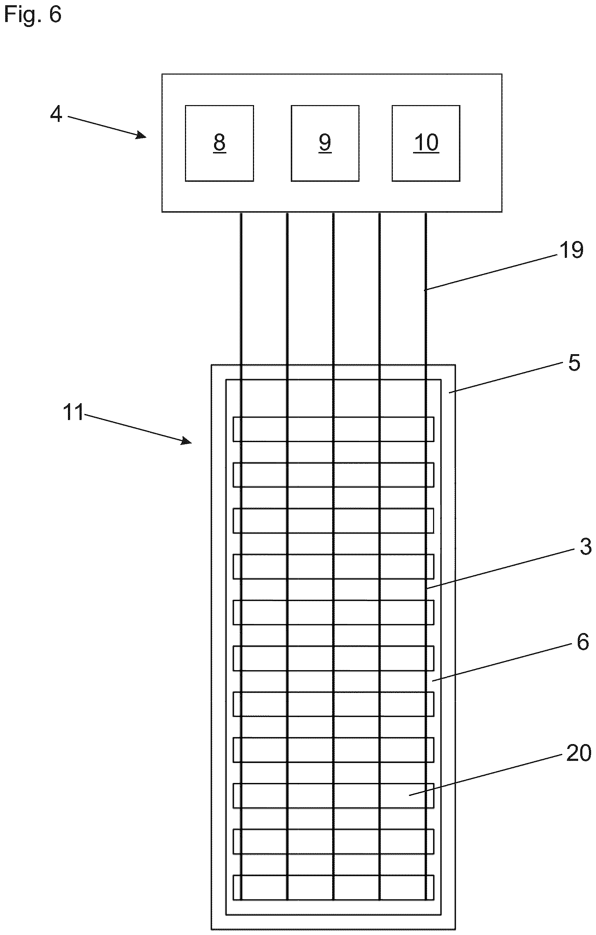

Der Feuchtesensor 1 kommuniziert dabei mittels eines Kommunikationsmoduls 10 (siehe dazu auf Fig. 6) mit dem mobilen Endgerät 14, auch welchem die Restfeuchtewerte, insbesondere in Form einer Restfeuchteverteilung, angezeigt und/oder weitergeleitet und/oder weiterverarbeitet werden können.

Rein schematisch sind die zumindest zwei, in diesem Fall fünf, Elektroden 3 eingezeichnet, die vertikal äquidistant (also äquidistant entlang einer vertikalen Achse) angeordnet sind, sodass Restfeuchtewerte in verschiedenen Tiefen des Baustoffs 2 über relative Widerstandsmessungen und/oder Impedanzmessungen und/oder Kapazitätsmessungen erfasst werden können.

Besonders bevorzugt können Vorrichtungen vorgesehen sein, die beim richtigen Positionieren des Feuchtesensors 1 behilflich sind und/oder die Beibehaltung der richtigen Positionierung sicherstellen.

Damit der Feuchtesensor 1 in der richtigen Tiefe im Baustoff 2 positioniert wird kann eine am Feuchtesensor 1 lösbar oder unlösbar befestigte Fahne 12 vorgesehen sein. Die Positionierung auf diese Weise kann bevorzugt bei "trockenen" Estrichen (oder von den Fließeigenschaften her ähnlichen Baustoffen 2) eingesetzt werden.

Alternativ oder zusätzlich kann ein (beispielsweise einklipsbarer und/oder fest mit dem Feuchtesensor 1 verbundener) Abstandhalter 13 vorgesehen sein, mittels dessen der Feuchtesensor 1 auf einem Untergrund positioniert werden kann. Der Abstandhalter 13 kann bei einem "nassen" Estrich (oder von den Fließeigenschaften her ähnlichen Baustoffen 2) sicherstellen, dass der Feuchtesensor 1 nicht mit der Zeit absinkt, sodass die Restfeuchtemessung nicht durch eine zeitlich veränderliche Position des Feuchtesensors 1 verfälscht wird.

Fig. 2 zeigt schematisch ein Ausführungsbeispiel eines erfindungsgemäßen Feuchtesensors 1. Dieser beinhaltet einen Trägerkörper 7 und eine an der Außenfläche des Trägerkörpers 7 angeordnete Messmembran 11.

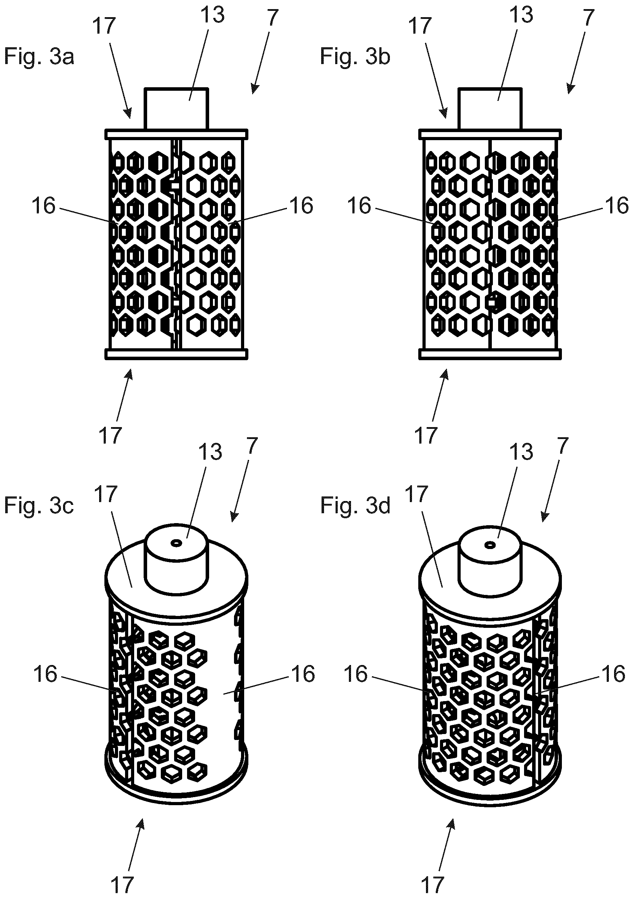

Für Ausführungsbeispiele des Trägerkörpers 7 wird auf die Figuren 3a bis 3d, 4a bis 4d sowie 5a bis 5d verwiesen.

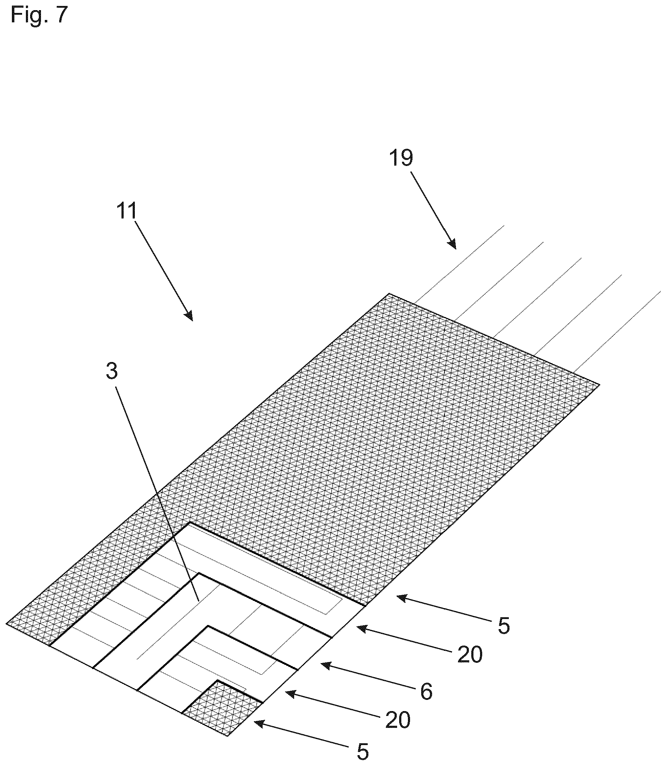

Für Ausführungsbeispiele der Messmembran 11 wird auf die Figuren 6 und 7 verwiesen.

Zu erwähnen ist, dass die Messmembran 11 in bevorzugten Ausführungsbeispielen eine Zwischenlage 6 mit aufgestickten Elektroden 3 beinhaltet, wobei die Zwischenlage 6 beidseitig mit semi-permeablen Membranen 5 laminiert ist.

Die Messmembran 11 ist im Ausführungsbeispiel nach Fig. 2 an einem Ende an einer vertikalen Öffnung des Trägerkörpers 7 eingehängt, dann um die zylindrische Außenfläche des Trägerkörpers 7 herumgewickelt und mit dem anderen Ende in die vertikale Öffnung gesteckt. Anschließend ist Messmembran 11 mittels einer Verklebung 15 in der vertikalen Öffnung fixiert.

Auf diese Weise wird die Messmembran 11 sicher an der Außenfläche des Trägerkörpers 7 gehalten und die parallele und vertikal äquidistante Anordnung der Elektroden 3 wird sichergestellt.

Natürlich sind auch andere Ausführungen denkbar. Beispielsweise könnte die Messmembran 11 nur teilweise umlaufend um den Trägerkörper 7 angeordnet sein und/oder im Innen im Trägerkörper geklemmt sein.

Grundsätzlich denkbar sind Ausführungsformen, wobei die Lagen der semi-permeablen Membran 5 und die dazwischen angeordneten Elektroden ohne einen Trägerkörper 7 im Baustoff 2 angeordnet werden. Bevorzugt sind aber Ausführungsformen mit einem Trägerkörper 7.

Außerdem zeigt das Ausführungsbeispiel nach Fig. 2, dass ein Abstandhalter 13 auch an einer Oberseite des Feuchtesensors 1 verwendet werden kann und zwar alternativ oder zusätzlich zum Abstandhalter 13 an der Unterseite und zur Fahne 12.

Fig. 3a bis 3d zeigen schematisch eine Ausführungsform eines Trägerkörpers 7 in zwei Aufsichten (Fig. 3a und 3b) und zwei perspektivischen Darstellungen (Fig. 3c und 3d).

Im vorliegenden Ausführungsbeispiel ist der Trägerkörper 7 mehrteilig aufgebaut. Es sind zwei Halbschalen 16 sowie jeweils eine obere und eine untere Abdeckung 17 vorhanden. Insgesamt ergibt sich eine zylindrische Grundform des Trägerkörpers 7 mit kreisförmiger Grundfläche.

Die Außenfläche des Trägerkörpers 7, an welcher die Messmembran 11 angeordnet werden kann, ist mit einer Wabenstruktur versehen, mittels welcher eine relativ geringe Masse des Trägerkörpers 7 bei hoher mechanischer Belastbarkeit erzielt wird.

In den Fig. 3a und 3c ist jeweils die vertikale Öffnung sichtbar, welche zum Einhängen der Messmembran 11 und für die anschließende Verklebung 15 der Messmembran 11 verwendet wird.

Verklebungen 15, Versiegelungen (beispielsweise mittels eines Kunststoffs) und/oder Verschweißungen können bevorzugt auch an den Fugen zwischen den anderen Bauteilen, d.h. insbesondere zwischen den Abdeckungen 17 und dem Rest des Feuchtesensors 1, verwendet werden, um das Eindringen von unerwünschten Fremdstoffen in das Innere des Feuchtesensors 1 und/oder des Trägerkörpers 7 zu verhindern.

Die Abdeckungen 17 sind in diesem Ausführungsbeispiel mit einem Kunststoff auf Basis von Methylmethacrylat versiegelt.

Insgesamt ist das Innere des Trägerkörpers 7 dadurch versiegelt, sodass nur Feuchtigkeit durch die semi-permeable Membran 5 zu den Elektroden 3 und in das Innere des Trägerkörpers 7 gelangen kann, nicht jedoch unerwünschte Fremdstoffe.

Der kreiszylindrische Trägerkörper 7 in diesem Ausführungsbeispiel weist eine Höhe von 50 mm und einen Durchmesser von 32 mm auf.

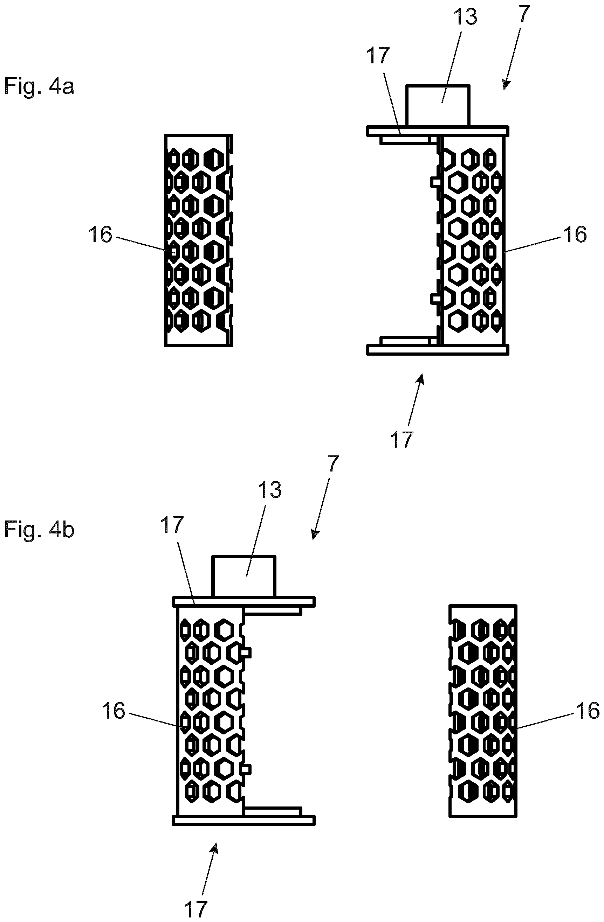

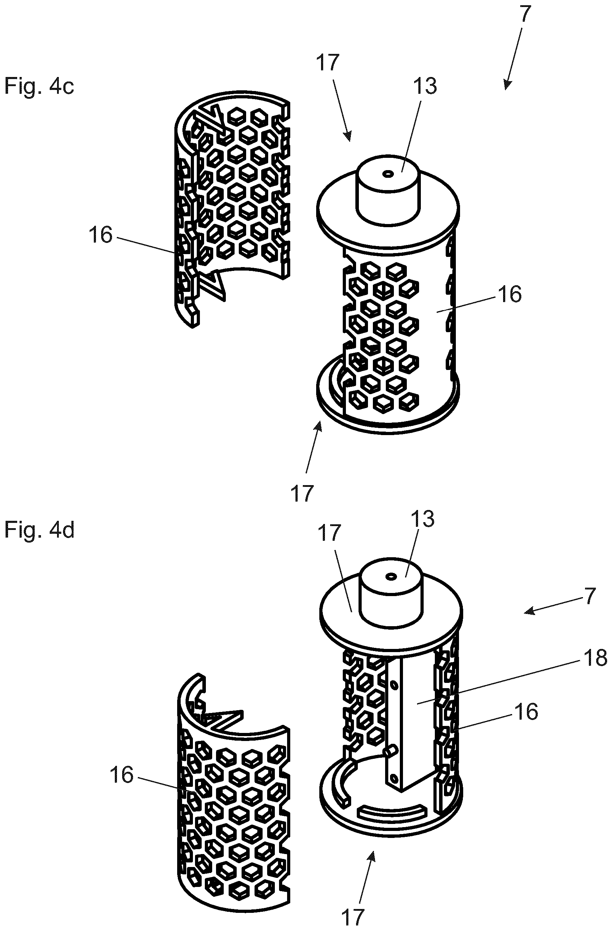

In den Figuren 4a bis 4d ist der Trägerkörper aus Fig. 3a bis 3d in einer teilweise zusammengebauten Anordnung, wobei eine der Halbschalen 16 mit den Abdeckungen 17 verbunden ist und die andere von der dadurch geschaffenen Anordnung gelöst ist. In diesem Zustand kann die Messmembran 11 wie beschrieben eingehängt werden, die bis dahin lose Halbaschale 16 kann befestigt werden und die Messmembran 11 kann um den zylindrischen Körper gewickelt werden.

Zu erwähnen ist, dass die in den Fig. 4a bis 4d dargestellte Anordnung der Halbschale 16 mit den damit in Kontakt stehenden Abdeckungen 17 in bevorzugten Ausführungsformen einstückig hergestellt werden kann, bevorzugt durch ein additives Fertigungsverfahren, bspw. Kunststoff-3D-Druck.

In Fig. 4d ist ein Montagekörper 18 erkennbar, der im Inneren einer der Halbschalen 16 und im assemblierten Zustand im Inneren des Trägerkörpers 7 angeordnet ist. Der Montagekörper 18 kann dazu verwendet werden, die Messelektronik 4, beispielsweise in Forme eines "printed circuit boards" (PCB), beispielsweise mittels Schrauben, besonders bevorzugt genau zwei Schrauben, im Trägerkörper 7 zu montieren.

Dabei kann es bevorzugt vorgesehen sein, dass die in die Halbschale 16 eingehängte Messmembran 11 durch die Messelektronik 4 klemmend gehalten wird. Wie erwähnt, kann die alternativ oder zusätzlich zur Verklebung 15 vorgesehen sein. Für ein Ausführungsbeispiel der Messelektronik sei auf Fig. 6 verwiesen.

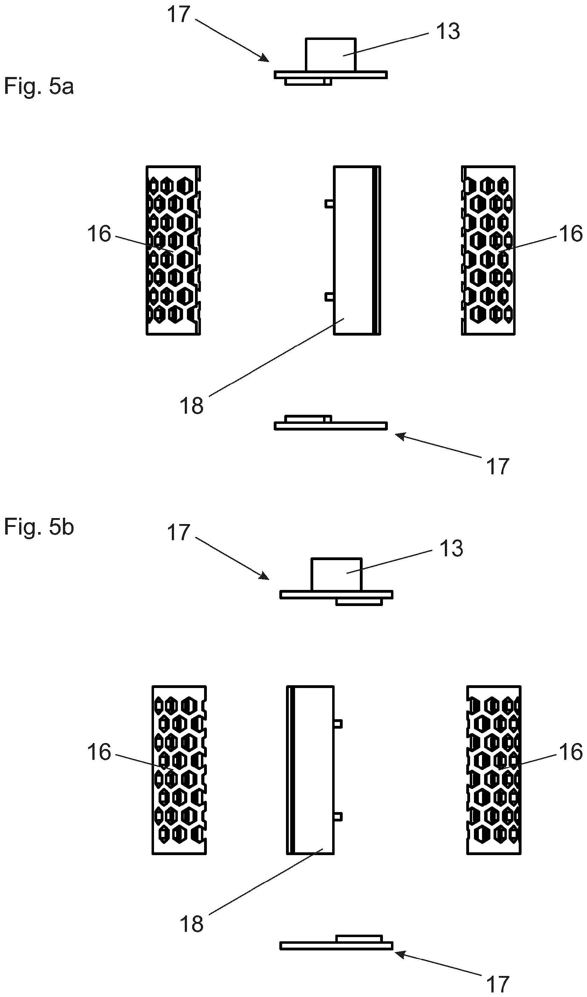

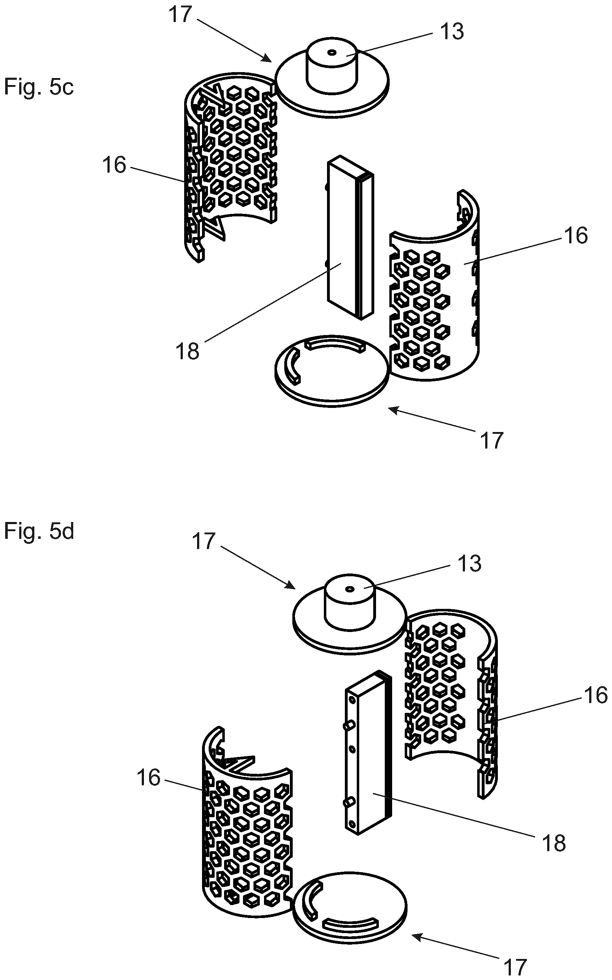

Die Figuren 5a bis 5d sind schematische Explosionsdarstellungen zum Ausführungsbeispiel aus den Figuren 3a bis 3d.

Wie bereits erwähnt können die Abdeckungen 17 separat oder einstückig mit einer der Halbschalen 16 hergestellt werden.

Fig. 6 zeigt schematisch ein Ausführungsbeispiel einer Messmembran 11 zusammen mit einer Messelektronik 4.

Die Messmembran 11 besitzt einen Lagenaufbau, der näher in Verbindung mit Fig. 7 beschrieben wird. Vorhanden sind zwei außenliegende Lagen semi-permeabler Membran 5, zwischen der eine Zwischenlage 6 mit aufgestickten Elektroden 3 angeordnet sind. Klebeschichten 20 können verwendet werden, um die einzelnen Lagen miteinander zu verbinden.

Die Messelektronik 4 ist über Anschlusskabel 19 elektrisch mit den Elektroden 3 verbunden. Mithin werden nur jene Teil der Elektroden 3 im Sinne der Erfindung auch als Elektroden verstanden, welche auch zwischen den Lagen der semi-permeablen Membran 5 angeordnet sind.

Die Anschlusskabel 19 können bevorzugt über Klemmen (nicht dargestellt, beispielsweise mit einer Klemmfläche von 4 mm mal 5 mm) an der Messelektronik angeschlossen sein.

Die Messelektronik 4 ist dazu ausgebildet, die Realteile der Impedanzen zwischen den einzelnen Elektroden 3 zu messen, welche für die Restfeuchte, insbesondere Restfeuchteverteilung, indikativ sind.

Zusätzlich ist im vorliegenden Ausführungsbeispiel ein Temperatursensor 8 vorgesehen, dessen Messwerte dazu verwendet werden können, die Messwerte Impedanzmessung zu kalibrieren, da die Impedanz zwischen den Elektroden neben einer Abhängigkeit von der Feuchtigkeit eine Abhängigkeit von der Temperatur aufweist.

Zusätzlich ist im vorliegenden Ausführungsbeispiel ein Luftfeuchtesensor 9 vorgesehen, dessen Messwerte als Redundanz zu den Messwerten der Impedanz zwischen den Elektroden 3 herangezogen werden können.

Sämtliche erwähnten Messwerte können über das Kommunikationsmodul 10 an ein Empfangsgerät, beispielsweise ein mobiles Endgerät 14, übermittelt werden. Alternativ kann auch bereits ein Teil der - mit an sich bekannten Algorithmen durchzuführenden - Datenauswertung auf der Messelektronik 4 geschehen und es können dann alternativ oder zusätzlich die Ergebnisse der Datenauswertung an das Empfangsgerät übermittelt werden.

Wie erwähnt, kann die Messelektronik 4 in an sich bekannter Weise als "printed circuit board" (PCB) realisiert werden. In solchen Ausführungsbeispielen kann die Messelektronik 4 auch als Platine bezeichnet werden.

Die Platine kann besonders bevorzugt so ausgeführt sein, dass eine Seite nur zur Kontaktierung der Anschlusskabel 19 dient und die andere Seite mit verschiedenen Elektronikbauteilen bestückt ist, um Kurzschlüsse zu vermeiden.

Die Messelektronik 4 ist in diesem Ausführungsbeispiel batteriebetrieben.

Der Lagenaufbau der Messmembran 11 ist, wie erwähnt, in Fig. 7 näher dargestellt.

Außen liegen jeweils zwei Lagen semi-permeabler Membran 5.

Die dazwischen angeordnete Zwischenlage 6 ist in diesem Ausführungsbeispiel ein Polyester-Baumwoll-Mischgewebe, welches eine sehr gute Saugfähigkeit aufweist.

Die Lagen der semi-permeablen Membran 5 sind mit der Zwischenlage 6 jeweils durch Klebeschichten 20 miteinander verbunden. Es ist dabei zu erwähnen, dass die Klebeschichten 20 nur teilflächig - anders formuliert: nicht vollflächig - zwischen der Zwischenlage 6 und der jeweiligen Lage semi-permeabler Membran 5 angeordnet sind, sodass der Durchtritt von Feuchtigkeit von der semi-permeablen Membran 5 zur Zwischenlage 6 und/oder den Elektroden 3 nicht gehemmt wird.

Bei der semi-permeablen Membran 5 handelt es sich im vorliegenden Ausführungsbeispiel um Zwei-Lagen-Laminat der Firma Toray mit dem Namen Dermizax. Wesentlicher Bestandteil der Membran 5 ist Polyurethan.

Die Elektroden 3 sind in diesem Ausführungsbeispiel auf die Zwischenlage 6 aufgestickt.

Der Abstand zwischen den Elektroden 3 beträgt in diesem Ausführungsbeispiel 8 mm.

Die Elektroden 3 aus diesem Ausführungsbeispiel als Kammelektrode mit mehreren Kanälen aufgefasst werden.

Die Elektroden 3 bestehen in diesem Ausführungsbeispiel aus Edelstahl, wobei natürlich grundsätzlich auch andere leitfähige Materialien eingesetzt werden können. Bevorzugt können Elektroden 3 aus Edelstahl aufgrund ihrer Korrosionsbeständigkeit sein.

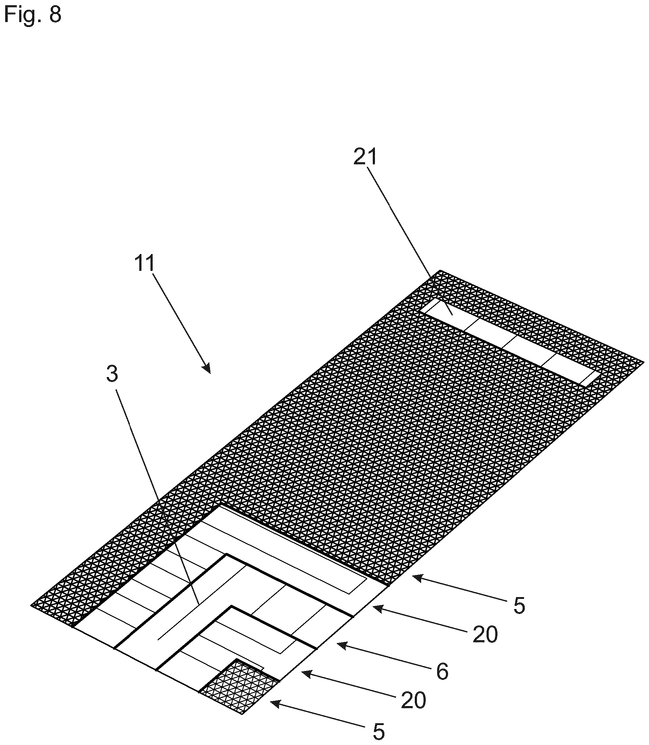

Fig. 8 zeigt ein zu Fig. 7 alternatives Ausführungsbeispiel einer Messmembran 11.

Statt Anschlusskabeln 19 ist dabei eine Ausnehmung 21 in einer der Lagen der semi-permeablen Membran 5 vorgesehen, wodurch die Elektroden 3 im Bereich der Ausnehmung 21 zugänglich sind.

Die Messmembran 11 gemäß der Ausführungsform nach Fig. 8 kann im Bereich der Ausnehmung 21 bevorzugt zwischen die Platine, welche die Messelektronik 4 realisiert (oder Teil derselben ist), und den Montagekörper 18 geklemmt werden. Durch geeignete Kontakte an der Messelektronik 4 kann durch diese Klemmung die elektrische Kontaktierung der Elektroden 3 mit der Messelektronik 4 realisiert werden.

Im Übrigen ist die Ausführungsform gemäß Fig. 8 analog zu jener aus Fig. 7, was insbesondere am visualisierten Lagenaufbau rechts unten zu erkennen ist.

In anderen Ausführungsformen könnte beispielsweise nur eine Lage der semi-permeablen Membran 5 zum Einsatz kommen, wobei die semi-permeable Membran 5 beispielsweise an der Außenfläche des Trägerkörpers 7 angeordnet ist und die Elektroden 3 im Inneren / Innenraum des Trägerkörpers 7 angeordnet sind. Beispielsweise könnten die Elektroden 3 dafür an der Innenseite des semi-permeablen Membran 5 angeordnet sein, sodass sich vorzugweise solche Ausführungsformen von den in den Figuren dargestellten Ausführungsformen im Wesentlichen nur dadurch unterscheiden, dass die innere Lage der semi-permeablen Membran 5 entfällt

### Legende

### zu den Hinweisziffern:

- 1: Feuchtesensor
- 2: Baustoff
- 3: Elektroden
- 4: Messelektronik
- 5: semi-permeablen Membran
- 6: Zwischenlage
- 7: Trägerkörper
- 8: Temperatursensor
- 9: Luftfeuchtesensor
- 10: Kommunikationsmodul
- 11: Messmembran
- 12: Fahne
- 13: Abstandhalter
- 14: mobiles Endgerät
- 15: Verklebung
- 16: Halbschalen
- 17: Abdeckungen
- 18: Montagekörper
- 19: Anschlusskabel
- 20: Klebeschichten
- 21: Ausnehmung

## Patentansprüche

1. Feuchtesensor geeignet zur Restfeuchtemessung in einem Baustoff (2), insbesondere einem Estrich oder einem Beton, mit zumindest zwei Elektroden (3), bevorzugt zumindest drei Elektroden (3), und einer Messelektronik (4) zum Messen einer für die Restfeuchte indikativen Impedanz und/oder eines für die Restfeuchte indikativen Widerstands und/oder einer für die Restfeuchte indikativen Kapazität zwischen den zumindest zwei Elektroden (3), **dadurch gekennzeichnet, dass** die zumindest zwei Elektroden (3) innerhalb zumindest einer semi-permeablen Membran (5) angeordnet sind, wobei die zumindest eine semi-permeable Membran (5) eine höhere Durchlässigkeit für Wasserdampf aufweist als für Fremdstoffe, insbesondere größere Moleküle als Wasser und/oder Alkali-Ionen, welche die Messung der Impedanz und/oder des Widerstands und/oder der Kapazität verfälschen können.

2. Feuchtesensor nach Anspruch 1, wobei die zumindest zwei Elektroden (3) zwischen zwei Lagen der zumindest einen semi-permeablen Membran (5) angeordnet sind und vorzugsweise zwischen den Lagen der zumindest einen semi-permeablen Membran (5) eine textile Zwischenlage (6) angeordnet ist, in welche die zumindest zwei Elektroden (3) eingearbeitet sind.

3. Feuchtesensor nach einem der vorhergehenden Ansprüche, wobei die zumindest zwei Elektroden (3) als Drahtelektroden ausgebildet sind und/oder Garnelektroden ausgebildet sind und/oder aus Edelstahl bestehen.

4. Feuchtesensor nach Anspruch 2 und 3, wobei die zumindest zwei Elektroden (3) auf die textile Zwischenlage (6) durch Sticken, Weben, Wirken, Stricken, Nähen, Steppen und/oder Drucken auf die Zwischenlage aufgebracht sind.

5. Feuchtesensor nach einem der vorhergehenden Ansprüche, wobei ein Trägerkörper (7) vorgesehen ist und die zumindest eine semi-permeablen Membran (5), vorzugsweise samt zumindest zwei Elektroden (3), an einer Außenfläche des Trägerkörpers (7) angeordnet sind, wobei die Außenfläche des Trägerkörpers (7) bevorzugt eine Wabenstruktur beinhaltet.

6. Feuchtesensor nach Anspruch 5, wobei die Messelektronik (4) in einem Inneren des Trägerkörpers (7) angeordnet ist.

7. Feuchtesensor nach 5 oder 6, wobei der Trägerkörper (7) eine zylindrische Grundform, vorzugsweise mit kreisförmiger Grundfläche, aufweist.

8. Feuchtesensor nach einem der Ansprüche 5 bis 7, wobei offene Seiten, bevorzugt obere und/oder untere Deckflächen, des Trägerkörpers (7) mittels eines Kunststoffs versiegelt sind.

9. Feuchtesensor nach einem der vorhergehenden Ansprüche, wobei mehr als zwei, bevorzugt fünf, Elektroden (3) vorgesehen sind.

10. Feuchtesensor nach einem der vorhergehenden Ansprüche, wobei die zumindest zwei Elektroden (3) entlang einer Achse, bevorzugt äquidistant, beabstandet sind.

11. Feuchtesensor nach einem der vorhergehenden Ansprüche, wobei ein Temperatursensor (8) und/oder ein Luftfeuchtesensor (9) vorgesehen ist, welcher bevorzugt im Inneren des Trägerkörpers (7) angeordnet ist.

12. Feuchtesensor nach einem der vorhergehenden Ansprüche, wobei ein Kommunikationsmodul (10) geeignet zur drahtlosen Kommunikation von Messwerten und/oder Restfeuchtewerten vorgesehen ist, welches bevorzugt im Inneren des Trägerkörpers (7) angeordnet ist.

13. Feuchtesensor nach zumindest den Ansprüchen 2 und 5, wobei in zumindest einer der Lagen der zumindest einen semi-permeablen Membran (5) eine Ausnehmung (21) vorhanden ist und die zumindest zwei Elektroden (3) mit einer Platine kontaktiert sind, indem die zumindest zwei Elektroden (3) samt der zumindest einen semi-permeablen Membran (5) im Bereich der Ausnehmung (21) zwischen die Platine und den Trägerköper (7), vorzugsweise im Bereich eines Montagekörpers (18) des Trägerkörper (7), geklemmt ist.

14. Anordnung aus einem Baustoff (2), insbesondere ein Estrich oder ein Beton, und zumindest einem Feuchtesensor (1) nach einem der vorhergehenden Ansprüche.

15. Anordnung nach Anspruch 14 mit einem Feuchtesensor (1) nach Anspruch 10, wobei die Achse, entlang der die zumindest zwei Elektroden (3) beabstandet sind, normal zu einer Oberfläche des Baustoffs (2) ausgerichtet ist.

16. Verfahren zum Messen einer Restfeuchte in einem Baustoff (2), insbesondere einem Estrich oder einem Beton, wobei
- zumindest ein Feuchtesensor (1) mit zumindest zwei Elektroden (3), die innerhalb zumindest einer semi-permeablen Membran (5) angeordnet sind, verwendet wird, wobei die zumindest eine semi-permeable Membran (5) eine höhere Durchlässigkeit für Wasserdampf aufweist als für Fremdstoffe, insbesondere größere Moleküle als Wasser und/oder Alkali-Ionen, welche die Messung einer Impedanz und/oder eines Widerstands und/oder einer Kapazität verfälschen können,
- der zumindest eine Feuchtesensor (1) in den Baustoff (2) eingegossen oder eingesetzt wird und
- die für die Restfeuchte indikative Impedanz und/oder en für die Restfeuchte indikativer Widerstand und/oder die für die Restfeuchte indikativen Kapazität zwischen den zumindest zwei Elektroden (3) gemessen wird.

17. Verwendung eines Feuchtesensors nach einem der Ansprüche 1 bis 13 und/oder einer Anordnung nach Anspruch 14 oder 15 bei einem Verfahren nach Anspruch 16.
